# EUROPEAN PATENT APPLICATION

(11) **EP 2 892 022 A1**
(43) Date of publication of application: **08.07.2015**
(21) Application number: 13832525.3
(22) Date of filing: 30.08.2013
(51) Int. Cl.: G06Q 50/22

(54) **METHOD AND APPARATUS FOR PERSONAL MEDICAL TREATMENT USING MOBILE TERMINAL**

(30) Priority: 31.08.2012 KR 20120096330
(71) Applicant: Samsung Electronics Co., Ltd., Suwon-si, Gyeonggi-do 443-742 (KR)
(72) Inventor: JUNG, Haemoon, Suwon-si Gyeonggi-do 441-100 (KR)
(74) Representative: Nederlandsch Octrooibureau
(86) International application number: PCT/KR2013/007815
(87) International publication number: WO 2014/035182

(57) **Abstract**

A method for enabling a terminal to provide medical information, according to one embodiment of the present specification so as to perform a particular task, comprises the steps of: receiving a symptom input of a user; suggesting a possible disease on the basis of the inputted symptom; outputting the suggested disease on a display unit; and receiving a disease selection input of the user on the basis of the suggested disease. The present invention according to one embodiment of the present specification can provide a method and an apparatus for operating the terminal which can effectively suggest information such as a selection of medical subjects, a selection of hospitals, and a selection of pharmacies and the like when the user recognizes an abnormal symptom, and enable the user to easily manage health care by storing the information related therewith and suggesting medical information on the basis of stored information.

## Description

### TECHNICAL FIELD

The present invention relates to a method and apparatus that: allows a user to input his/her symptoms to a mobile terminal; recommends clinics, hospitals, and pharmacies, referring to a variety of elements, such as symptoms, clinic records, epidemic dieses, etc.; manages the user's health care; etc. More particularly, the present invention relates to a mobile terminal of managing a user's health care information, associated with a hospital server, a pharmacy server and an emergency aid server.

### BACKGROUND ART

When people are sick or injured, they must directly determine a type of their diseases based on their knowledge, select a corresponding clinic hospital and then visit it. If people need to obtain their clinic records, etc., they must access web pages of hospitals or make a wired phone call thereto.

In recent years, the development of mobile communication technology has led to develop telemedicine. It is, however, limited to be used. For example, telemedicine is used only after a certain level of diagnosis has been performed. Telemedicine is often used to take an action after the examination and treatment has online first been performed.

Therefore, a method and apparatus is required that helps users to receive examination and treatment by mobile terminals easily accessible by them and recommends hospitals, pharmacies, etc., to them.

### DISCLOSURE OF INVENTION TECHNICAL PROBLEM

The present invention has been made in view of the above problems, and provides a method and apparatus that helps users who felt abnormal symptoms to receive diagnoses and recommends hospitals to receive examination and treatment and pharmacies to buy medicines according to the prescriptions to them. The present invention further provides a method and apparatus that increases the diagnostic accuracy for users by using information related to, for example, a user's clinical records, a user's medical history, a user's medical family history, etc., and performs post management after the examination and treatment. The present invention further provides a method and apparatus that integrally manages a database, associated with a hospital server, a pharmacy server, an emergency aid server, etc., and provides users with health care information optimized for them.

### SOLUTION TO PROBLEM

In accordance with an exemplary embodiment of the present invention, the present invention provides a method of providing medical information through a mobile device including: receiving a user's input symptoms; suggesting names of possible diseases based on the user's input symptoms; outputting the suggested disease names on a display unit; and receiving a user's input selecting a disease name from the suggested disease names.

In accordance with another exemplary embodiment of the present invention, the present invention provides a mobile device of providing medical information including: an input unit for receiving a user's input symptoms; a controller for suggesting names of possible diseases based on the user's input symptoms; and a display unit for outputting the suggested disease names on a display unit. The receiving unit receives a user's input selecting a disease name from the suggested disease names.

### ADVANTAGEOUS EFFECTS OF INVENTION

As described above, the method and apparatus according to an embodiment of the present specification: effectively provides a user who felt abnormal symptoms with information related to the selection of, for example, clinics, hospitals, pharmacies, etc.; stores the corresponding information; and provides his/her medical information based on the stored information, later, so that the user can easily manage his/her health care by his/her mobile terminal. The method and apparatus integrally manages information about users' health cares, associating with a hospital server, a pharmacy server, an emergency aid server, etc., and provides the users with health care information optimized for them, thereby enhancing use convenience.

### BRIEF DESCRIPTION OF DRAWINGS

The features and advantages of the invention will become more apparent from the following detailed description in conjunction with the accompanying drawings, in which:
FIG. 1 is a view of a medical information providing system according to an embodiment of the present invention;
FIG. 2 is a schematic block diagram of a mobile terminal according to an embodiment of the present invention;
FIG. 3 is a view that describes a method of inputting parts of the user's body where symptoms occurred to a mobile terminal according to an embodiment of the present invention;
FIG. 4 is a flow chart that describes a method of receiving medical services by using a mobile terminal according to an embodiment of the present invention;
FIG. 5 is a flow chart that describes a method of providing information about an emergency situation to a user by a mobile terminal according to an embodiment of the present invention; and
FIG. 6 is a flow chart that describes a method of providing medical information to a user based on a user's health information stored in a mobile terminal according to an embodiment of the present invention.

### MODE FOR THE INVENTION

Hereinafter, exemplary embodiments of the present invention are described in detail with reference to the accompanying drawings. Detailed descriptions of well-known functions and structures incorporated herein may be omitted to avoid obscuring the subject matter of the invention.

Although the drawings represent an embodiment of the invention, the drawings are not necessarily to scale and certain features may be exaggerated or omitted in order to better illustrate and explain the invention. The same reference numbers are used throughout the drawings to refer to the same or similar parts.

The present invention is described in detail referring to the accompanying drawings to describe mobile terminal according to embodiments of the present invention.

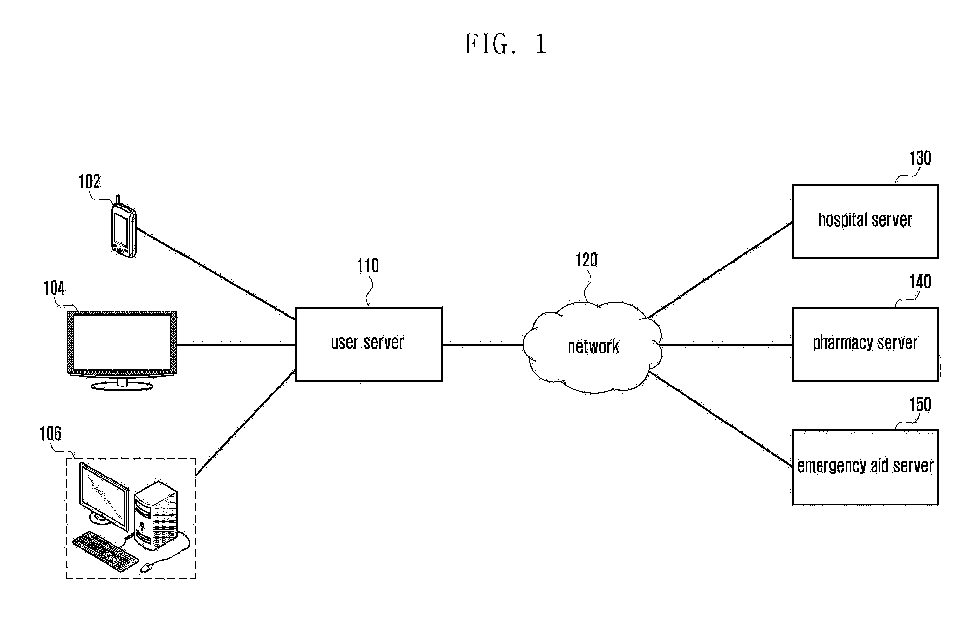

FIG. 1 is a view of a medical information providing system according to an embodiment of the present invention.

Referring to FIG. 1, the mobile terminal according to an embodiment of the present invention may include a mobile phone 102, a smart television (TV) 104 and a personal computer (PC) 106. It should be understood that mobile terminals that receives user's inputs and performs data communication with other devices may also be applied to the embodiments of the present invention.

The mobile terminals are connected to a user server 110. The user server 110 is connected to the mobile terminal and a network 120, transmits/receives information to/from the mobile terminal, and stores the information. The user server 110 shares the stored information with the mobile terminals. In another embodiment, the user server 110 includes an input unit and a display unit, so that it can receive a user's inputs via the input unit and display the results processed according to the inputs on the display unit.

The mobile terminals 102, 104 and 106 may access one or more of the following: hospital server 130, pharmacy server 140 and emergency aid server 150, via the network 120, without the user server 110, and may transmit/receive data to/from them. Access to the hospital server 130, pharmacy server 140 and emergency aid server 150 needs an authentication procedure to verify a user. The authentication method includes a method of confirming an identification and password and/or a method of using a public key certificate. It should be understood that the present invention may also employ well-known user authenticating methods.

The hospital server 130 stores one or more of the following: a user's clinical records, a user's medical family history, and a user's medical history. The hospital server 130 may also store hospital information about medical staff, medical equipment, sickrooms, etc. The hospital server 130 provides the stored information to the mobile terminals 102, 104 and 106. The hospital server 130 may also allow for offering telemedicine to users via their mobile terminals 102, 104 and 106 connected to the server 300. Telemedicine is performed in such a way that: a user (patient) inputs his/her health statuses to the input units of the mobile terminals 102, 104 and 106; the mobile terminal transmits the user's input health statuses to the hospital server 130; and the medical specialist makes a diagnosis or examination for the user. In an embodiment, after medical staff check a user's health statuses via a video call of the mobile phone 102 and make a diagnosis, they make a treatment for the user based on the diagnosis result. According to the diagnosis and treatment, they may write a prescription to the user. The prescription may be transmitted to one of the mobile terminals 102, 104 and 106 via the network 120.

The pharmacy server 140 stores one or more of the following: an item about medicines kept in pharmacies, an item about pharmacy business hour, and an item about a user's prescription history. In an embodiment, the pharmacy server 140 transmits one or more of the items to the mobile terminals 102, 104 and 106, and receives a user's prescription from the mobile terminals 102, 104 and 106. In an embodiment, when a pharmacy receives the user's prescription via the pharmacy server 140, it may make up the prescription. After that, the user visits the pharmacy and receives the prescribed medicines.

The emergency aid server 150 refers to servers related to emergency rescue mobilization of police, firefighters, etc. The emergency aid server 150 receives a user's report and manages the mobilization states. In an embodiment, when an emergency rescue service is provided to a user who is in an emergency situation, the user's mobile terminals 102, 104 and 106 transmit the stored information to the emergency aid server 150, so that it can check a user's health information fast and they can continue to rescue the user based on the checked user's health information.

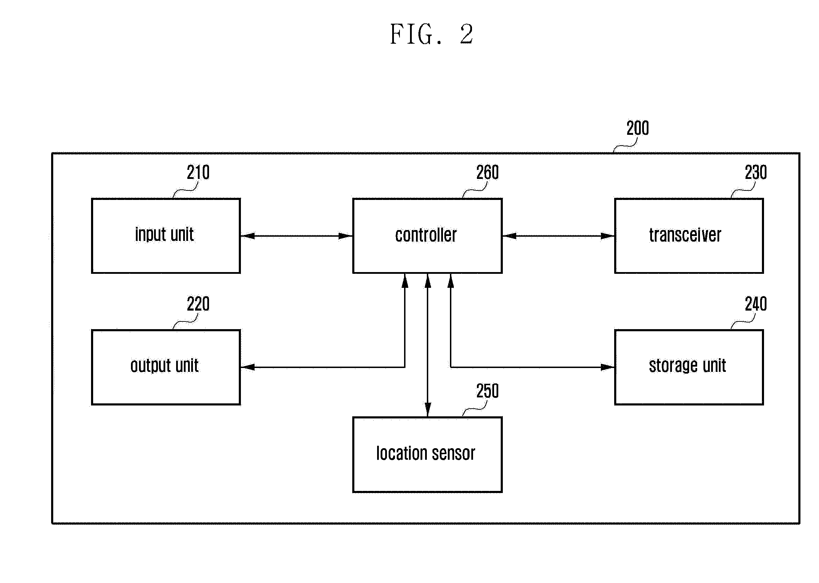

FIG. 2 is a schematic block diagram of a mobile terminal according to an embodiment of the present invention.

Referring to FIG. 2, the mobile terminal 200 includes an input unit 210 for receiving a user's inputs, an output unit 220 for displaying information received by the mobile terminal 200, a transceiver 230 for performing data transmission/reception, a storage unit 240 for storing input data, received data and data to be transmitted, a location sensor 250 for sensing a location of the mobile terminal 200, and a controller 260 for controlling the operations of the mobile terminal 200.

The input unit 210 includes one or more of the following: a keyboard, a touch pad, a microphone, a camera, a motion sensor, and a temperature sensor. The mobile terminal 200 determines the operations based on signals of the input unit 210. When a user needs telemedicine, the user's information required for diagnosis is input to the input unit 210. A user may input his/her symptoms to the input unit 210.

The output unit 220 outputs corresponding information under the control of the controller 260. The output unit 220 includes one or more of the following: a display, a speaker, a printer, a vibration motor, and a lighting emitting device. The output unit 220 provides corresponding information to users.

The transceiver 230 performs transmission/reception of information under the control of the controller 260. The transceiver 230 is connected to one or more of the following: a user server, a hospital server, a pharmacy server and an emergency aid server, and transmits/receives data to/from the connected server under the control of the controller 260.

The storage unit 240 stores information input to the mobile terminal 200, received information and information to be transmitted, under the control of the controller 260. The storage unit 230 includes flash memory or a hard disk. It should be understood that the storage unit 230 may also include media for storing information.

The location sensor 250 detects a location where the mobile terminal 200 is located. The location sensor 250 measures a location of the mobile terminal 200 by detecting GPS signals, signals during the mobile phone call, or Wi-Fi signals. When the location sensor 250 detects a location of the mobile terminal 200 where the user is in an emergency situation, it transmits it to the emergency aid server 150, so that the user can receive a corresponding service.

The controller 260 controls operations of the components in the mobile terminal 200 and also selects functions of the mobile terminal 200 based on the input/received information or information to be transmitted.

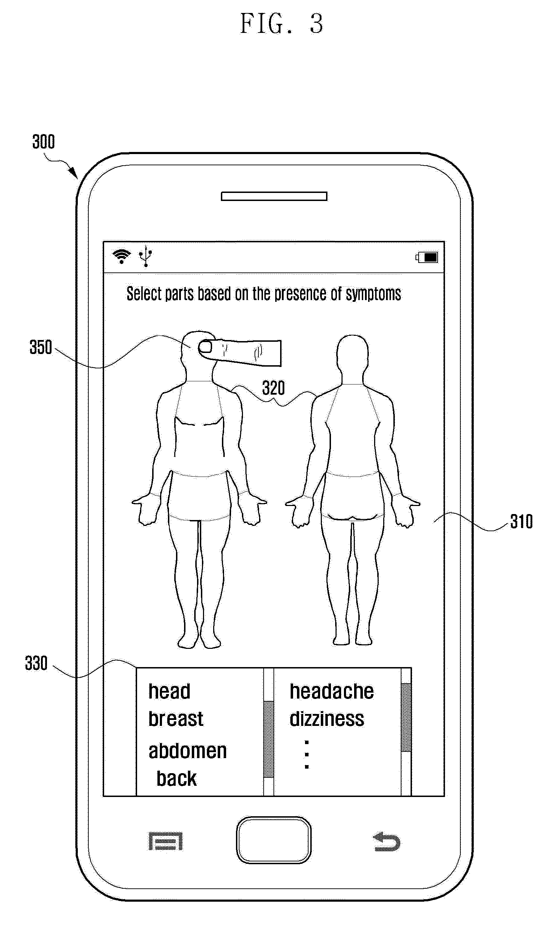

FIG. 3 is a view that describes a method of inputting parts of the user's body where symptoms occurred to a mobile terminal according to an embodiment of the present invention.

Referring to FIG. 3, the mobile terminal 300 includes a display unit 310. The display unit 310 displays corresponding information. The display unit 310 may be implemented with a touch screen that receives a touch input and displays corresponding information.

The mobile terminal 300 displays a human body model 320 used to receive a user's symptoms on the display unit 310. The human body model 320 is displayed in such a way that the area is separated by body parts so that the user can select corresponding parts based on his/her symptoms. The user selects one of the body parts, the head 350, and then corresponding symptom.

In another embodiment, the mobile terminal 300 allows a user to select his body part and symptoms from the list of body parts and symptoms 330. The user can receive a service, based on his/her selected symptoms, such as diagnosis, recommendation of hospitals and pharmacies, which will be described later.

When the user select a body part 350 form the human body model 320, the mobile terminal 300 may show the names of diseases related to the selected body part 350 on the list 330. In that case, the user may view additional information about symptoms about a corresponding disease on the display unit 310 according to his/her request and then select corresponding symptoms.

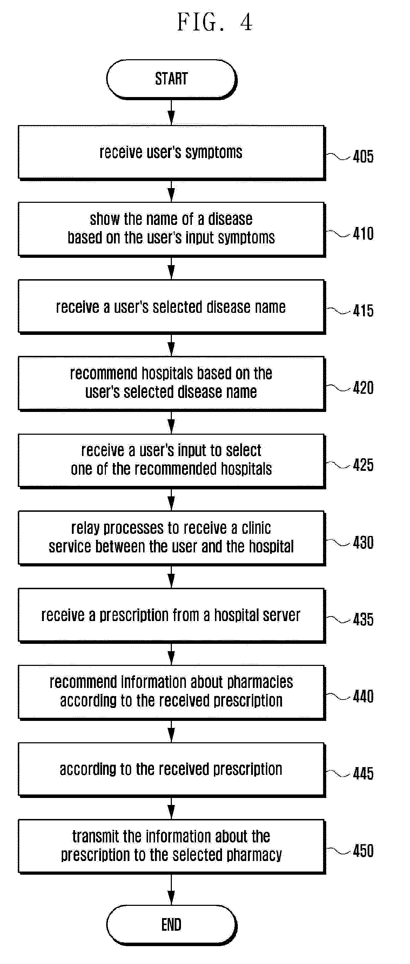

FIG. 4 is a flow chart that describes a method of receiving medical services by using a mobile terminal according to an embodiment of the present invention.

Referring to FIG. 4, the mobile terminal receives a user's symptoms (405). Inputting a user's symptoms to the mobile terminal may be performed by the method as described above referring to FIG. 3. In another embodiment, the symptom inputting method may also be implemented by inputting keywords. In another embodiment, the symptom inputting method may also be implemented in such a way as to show clinic categories, such as internal medicine, surgery, otorhinolaryngology, etc. and to receive a user's symptoms corresponding to the categories.

The mobile terminal shows the names of diseases based on the user's input symptoms (410). The mobile terminal may show the names of diseases based on one or more of the following: an item about a user's medical family history, an item about a user's clinical history, an item about a user's medical history, and an item about an epidemic disease. The items may be stored in the mobile terminal or received from other servers. The epidemic disease may be determined based on clinical histories of outpatients who have been treated for a disease showing the similar symptoms as it has showed within a preset period of time. The medical service informs a user of the names of diseases based on a number of information items. That is, it can provide the first diagnosis result of a relatively high level of reliability to the user.

The mobile terminal receives a user's selected disease name from the informed names of diseases (415).

The mobile terminal recommends one or more hospitals based on the user's selected disease name to the user (420). The one or more recommended hospitals may include clinics for the selected disease name. The recommendation of one or more hospitals may be performed based on one or more of the following: the present state of medical staff, medical equipment, reputations of users who have been examined and treated, the number of beds, the distance from the user, whether to provide telemedicine services, facilities such as parking services, etc.

For example, when a user selects enteritis as a disease name, a hospital may be recommended that is one of the hospitals with colonoscopy equipment, within a range of distance that the user has set from his/her current location, and has a good reputation. Since the service recommends hospitals and simultaneously provides the details about the hospitals to a user, the user can easily select a corresponding hospital.

The embodiment may be modified in such a way as not to include operations 410, 415 and 420. More specifically, the modification may be implemented in such a way that, when the mobile terminal receives a user's input symptoms, it automatically selects one or more possible names of diseases according to the symptoms and recommends hospitals related to the diseases to the user. This makes it possible to recommend a hospital to a user who has difficulty in selecting one of the disease names. The mobile terminal can inform the user of hospitals that can all treat a number of possible diseases.

The mobile terminal receives a user's input to select one of the recommended hospitals (425). The mobile terminal may also receive a user's input hospital besides the recommended hospitals.

The mobile terminal relays processes to receive a clinic service between the user and the hospital selected at operation 425 (430).

In an embodiment, the mobile terminal may book an appointment schedule for a clinic service, so that the user can visit the clinic hospital without failure.

In another embodiment, the mobile terminal may allow the user to receive a telemedicine service from a medical specialist of the selected hospital. The telemedicine is performed in such a way that the mobile terminal obtains information about a user's symptoms via the input unit and transmits it to a medical specialist, so that the medial specialist makes a diagnosis for the user.

The mobile terminal receives a prescription according to the diagnosis result (435). The prescription includes a list of medicines that the user will take and information how to take the medicines.

The mobile terminal recommends information about pharmacies according to the received prescription (440). The recommendation of information about pharmacies may be performed based on one or more of the following: the received prescription information, components of medicines, pharmacy companies of medicines, names of medicines, prices of medicines, reputations of pharmacies, and distances from the user's location to pharmacies.

In an embodiment, information about pharmacies is used to recommend one or more of the pharmacies that keep the same medicines as the companies and components of medicines written in the prescription, which can be easily accessible by the user from his/her current location, residence or hospital. In addition, since the service recommends pharmacies and simultaneously provides the details about the pharmacies to a user, it can increase in use convenience.

The mobile terminal receives a user's input to select a pharmacy (445). In an embodiment, the mobile terminal may receive a user's input to select one of the pharmacies recommended at operation 440 or a user's input to select another pharmacy.

The mobile terminal transmits the information about the prescription to the pharmacy selected at operation 445 (450). The information about the prescription may be transmitted to the selected pharmacy according to a user's option at operation 450. When the selected pharmacy receives the information about the prescription, it may prescribe corresponding medicines and immediately provide them to the user with his/her visit.

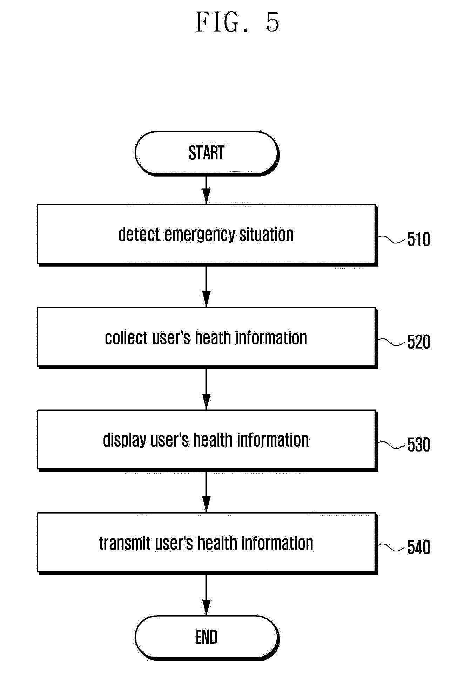

FIG. 5 is a flow chart that describes a method of providing information about an emergency situation to a user by a mobile terminal according to an embodiment of the present invention.

Referring to FIG. 5, the mobile terminal detects an emergency situation (510). The detection of an emergency situation may be flexibly performed according to a user's settings. In an embodiment, the mobile terminal may identify an emergency situation of a mobile terminal in such a way that it detects a strong impact, yet it doesn't detect a user's input within a preset period of time. In that case, the mobile terminal outputs a signal indicating that an emergency situation has been detected on the display unit and proceeds with the following process if it doesn't detect a user's additional input.

The mobile terminal collects a user's health information in order to respond to the identified emergency situation (520). The user's health information includes one or more of the following: an item about a user's medical family history, an item about a user's clinical history, an item about a medicating history, an item about medicines the user is currently taking, an item about allergic reactions to medications and an item about the blood type. The information items may be stored in the storage unit of the mobile terminal or transmitted from a hospital server or a pharmacy server.

The mobile terminal displays the user's collected health information on the display unit (530). The health information on the display unit may be selected according to a user's additional inputs. In an embodiment, when a user is in an emergency situation and a mobilized emergency rescuer applies a selection to the user's mobile terminal, the mobile terminal may display the user's collected health information on the display unit. This makes the emergency rescuer effectively handle the user's emergency situation.

The mobile terminal transmits the collected information to another server (540). The other server may be an emergency aid server, a hospital server or a pharmacy server. The transmission of the collected information to other server may be performed according to a user's option. The collected information may be transmitted to the server, along with the user's health information and the user's location information, simultaneously. Therefore, the hospital receives the user's information before the user arrives and can apply a proper service to the user immediately on arrival.

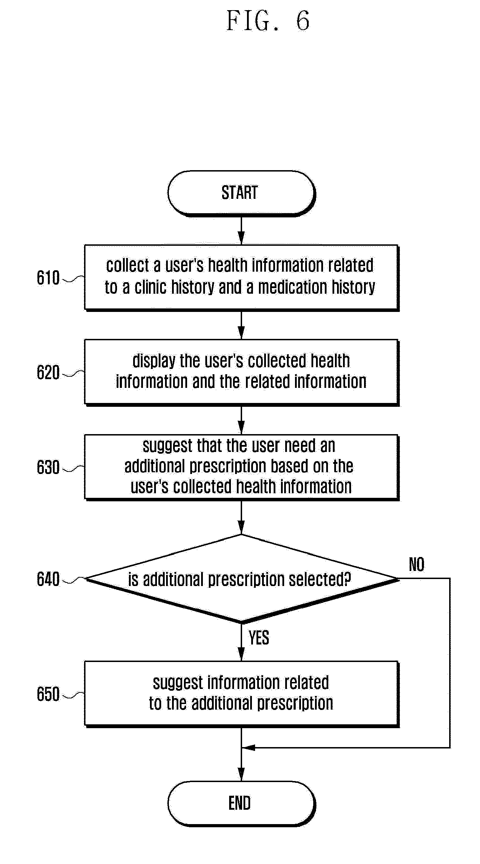

FIG. 6 is a flow chart that describes a method of providing medical information to a user based on a user's health information stored in a mobile terminal according to an embodiment of the present invention.

There may be a case where a user needs to receive a medical examination again after a first examination. If a medicine, the adverse reaction of which was reported, has been prescribed for a user, it is necessary to inform the user of information about a case where he/she takes it.

Referring to FIG. 6, the mobile terminal collects a user's health information related to a clinic history and a medication history (610). The mobile terminal may also collect a user's health information, including a user's medical family history.

The mobile terminal displays the user's collected health information on the display unit (620). The mobile terminal may display the user's collected health information on the display unit according to a user's option. The mobile terminal outputs the user's health information and informs the user of a clinic history or a medication history, thereby enhancing use convenience.

The mobile terminal suggests that the user need an additional prescription, based on the user's health information collected at operation 610 (630). The additional prescription may be suggested based on information about adverse reactions that may be caused according to medicines that the user is taking. In addition, if the user has a disease that may recur, a medical re-examination may also be suggested according to his/her clinic history.

The mobile terminal determines whether to select an additional prescription (640). When an additional prescription is selected at operation 640, the mobile terminal suggests information related to an additional prescription (650). This is performed in the same method as described above referring to FIG. 4.

Although exemplary embodiments of the invention have been described in detail above, it should be understood that many variations and modifications of the basic inventive concept herein described, which may be apparent to those skilled in the art, will still fall within the spirit and scope of the exemplary embodiments of the invention as defined in the appended claims.

## Claims

1. A method of providing medical information through a mobile device comprising:
receiving a user's input symptoms;
suggesting names of possible diseases based on the user's input symptoms;
outputting the suggested disease names on a display unit; and
receiving a user's input selecting a disease name from the suggested disease names.

2. The method of claim 1, wherein suggesting names of possible diseases comprises:
suggesting names of diseases corresponding to the user's input symptoms, based on one or more of the following: a user's medical family history, a user's clinic history, and a user's medication history and a treatment statistics of other users who have recently been treated.

3. The method of claim 1, wherein receiving a user's input symptoms comprises:
displaying the human body model corresponding to the user's body parts; and
receiving a user's input selecting a body part in the human body model, corresponding to his/her symptom.

4. The method of claim 1, further comprising:
recommending hospitals according to a disease corresponding to the user's selected disease name, based on one or more of the following: the present state of medical staff, medical equipment, reputations of users who have been examined and treated, the number of beds, the distance from the user, whether to provide telemedicine services, and facilities.

5. The method of claim 1, further comprising:
relaying a clinic process to treat the selected disease between the user and the hospital,
wherein relaying a clinic process comprises:
booking an appointment schedule for a clinic service or relaying a telemedicine service.

6. The method of claim 5, further comprising:
receiving information about a prescription according to the clinic process; and
recommending information about pharmacies abased on the received prescription information.

7. The method of claim 6, wherein recommending information about pharmacies comprises:
recommending pharmacies based on one or more of the following: components of medicines, pharmacy companies of medicines, names of medicines, prices of medicines, reputations of pharmacies, and distances from the user's location to pharmacies.

8. The method of claim 6, further comprising:
receiving a pharmacy selecting instruction based on the recommended pharmacy information; and
transmitting the prescription information to a server related to the selected pharmacy.

9. A mobile device of providing medical information comprising:
an input unit for receiving a user's input symptoms;
a controller for suggesting names of possible diseases based on the user's input symptoms; and
a display unit for outputting the suggested disease names on a display unit,
wherein the receiving unit receives a user's input selecting a disease name from the suggested disease names.

10. The mobile device of claim 9, wherein the controller suggests names of diseases corresponding to the user's input symptoms, based on one or more of the following: a user's medical family history, a user's clinic history, and a user's medication history and a treatment statistics of other users who have recently been treated.

11. The mobile device of claim 9, wherein:
the display unit displays the human body model corresponding to the user's body parts; and
the input unit receives a user's input selecting a body part in the human body model, corresponding to his/her symptom.

12. The mobile device of claim 9, wherein the controller recommends hospitals according to a disease corresponding to the user's selected disease name, based on one or more of the following: the present state of medical staff, medical equipment, reputations of users who have been examined and treated, the number of beds, the distance from the user, whether to provide telemedicine services, and facilities.

13. The mobile device of claim 9, further comprising:
a transceiver for transmitting/receiving data to/from other servers,
wherein:
the controller relays a clinic process to treat the selected disease between the user and the hospital; and
the clinic process comprises booking an appointment schedule for a clinic service or relaying a telemedicine service.

14. The mobile device of claim 13, wherein:
the transceiver receives information about a prescription according to the clinic process; and
the controller recommends information about pharmacies abased on the received prescription information.

15. The mobile device of claim 14, wherein the controller recommends pharmacies based on one or more of the following: components of medicines, pharmacy companies of medicines, names of medicines, prices of medicines, reputations of pharmacies, and distances from the user's location to pharmacies.
